# EUROPEAN PATENT SPECIFICATION

(11) **EP 0 285 263 B1**
(45) Date of publication and mention of the grant of the patent: **29.06.1994**
(21) Application number: 88301819.4
(22) Date of filing: 02.03.1988
(51) Int. Cl.: C12Q 1/34, C12Q 1/68, C12Q 1/70, A61K 31/70, A61K 45/00

(54) **Activated RNase L as a marker for virus infections**
Aktivierte RNase L als Markierung für Virus-Infektionen
RNase L activée comme marqueur pour des infections virales

(30) Priority: 03.03.1987 US 21372
(43) Date of publication of application: 05.10.1988
(73) Proprietor: HEM PHARMACEUTICALS CORP., Rockville, MD 20852 (US)
(72) Inventor: Carter, William A., Birchrunville, PA (US)
(74) Representative: Votier, Sidney David

(56) References cited:
- EP-A- 0 113 162
- EP-A- 0 213 921
- LANCET, June 1987, GB; W.A. CARTER et al., pp. 1286-1292#
- CHEMICAL ABSTRACTS, vol. 98, no. 7, 14 February 1983, Columbus, OH (US); B.R.G. WILLIAMS et al., p. 503, no. 51575w#
- NUCLEIC ACIDS RESEARCH, vol. 9, No. 7, 1981, Arlington, VA (US); D.H. WRESCHNER et al., pp. 1517-1581#
- JOURNAL OF INFECTIOUS DISEASES, vol. 152, no. 3, 03 September 1985, Chicago, IL (US); O.T. PREBBLE et al., pp. 457-465#
- BIOLOGICAL ABSTRACTS, vol. 34, 1988; K. KARIKO et al.#
- J.D. WATSON et al., "Molecular Biology of the Gene" 1987, Benjamin/Cummings Publishing Co., Menlo Park, CA (US)#

## Description

### GENERAL BACKGROUND ON AIDS

AIDS (Acquired Immunodeficiency Syndrome) represents a major public health threat of the Twentieth Century. Because of its biologically variable nature, its subtle means of spread from individual to individual, and its "latent" disease period which defies even the ready detection of its very presence, AIDS has rapidly evolved into an epidemiologic dilemma of unprecedented proportions. Indeed, even with the considerable resources already deployed to increase diagnostic capabilities, the available technologies still fall well short of the necessary requirements to gain firm control of this problem within any section of the population, e.g., see Proceedings of Workshop entitled "Experience with HTLV-III Antibody Testing, an Update on Screening, Laboratory and Epidemiological Correlations", sponsored by the Center for Drugs and Biologics, FDA, National Institutes of Health, and Centers for Disease Control, held on July 31, 1985, at the National Institutes of Health, Bethesda, Maryland; see also, Budiansky, S. in Nature, volume 316, page 96, July 11, 1985.

Several different designations for the human immunodeficiency virus (HIV) virus exist; LAV is the designator for the HIV virus isolated at the Pasteur Institute, Paris, France, and HTLV-III (human T-cell lymphotropic virus) is the designator for the HIV virus isolated at the National Institute of Health, Bethesda, Maryland, U.S.A. Frequently in this text, the HIV virus will be referred to generically or designated HTLV III or LAV or HIV without intending to differentiate between them. Furthermore, the term "HIV" in the specification includes any and all other viruses which may be associated with producing AIDS, whether yet isolated or not. HIV virus in the singular is meant to include any of the HIV types regardless of their genomic contents. Similarly, AIDS symptoms will be referred to generically to include symptoms caused by the HIV (as defined above), ARC or AIDS related complex, lymphadenopathy syndrome (LAS), and other viruses causing substantially similar clinical conditions.

Current therapeutic considerations have taken one of two approaches: immunological (vaccine production) or direct attacks on the virus itself (antiviral therapy). While vaccine production initially appeared extremely promising, at least in theory, new knowledge of the viral composition has significantly undermined this promise; namely, the virus apparently readily mutates or changes its basic biochemical structure such that critical viral antigenic "determinants" -- necessary for vaccines to be effective -- readily undergo mutation or change. For example, HTLV-III isolates in California, Maryland and England have recently been found to be significantly different from each other when their genomic content(s) has been rigorously analyzed. The implication of such studies is that vaccination against HIV will not have widespread lasting benefits such as the conclusively beneficials/durable results historically characteristic of most other vaccines, such as those directed against polio virus, measles virus, etc.

In the second, or direct antiviral approach, other scientists have tested several compounds including AZT, HPA-23, suramin, ribavirin, interferon and fascarnet. These compounds have been introduced into either the laboratory or clinical studies with limited or qualified evidence of therapeutic success to date. Indeed, consistent evidence of high-toxicity and/or severe side-effects has been reported in nearly every instance (e.g., see summary prepared by M. Clark, Newsweek, page 71, August 5, 1985; also C. Wallis, Time, pages 40-47, August 12, 1985). None of these drugs are indeed new or able to be selectively directed against the underlying disorder; namely, multiplication of HIV in certain cells. For example, HPA-23 is a combination of heavy metals (reminiscent of the use of arsenic to treat venereal diseases in the 1920's, or pre-penicillin era); suramin is actually an anti-parasitic (sleeping sickness) compound and fascarnet is an anti-herpes virus compound. The latter two compounds may inhibit an HIV-related enzyme termed "reverse transcriptase", but there is little evidence that such an enzymatic inhibition, in fact, would result in any significant therapeutic improvement or disease prevention/amelioration. AZT may be associated with a slight prolongation of life in AIDS victims, but it is highly toxic and does not eradicate HIV (R. Yarchoan et al, Lancet, March 15, p. 575, 1986; R.E. Chaisson et al, New England Journal of Medicine, volume 315, p. 1611, 1986).

### Progressive Features of AIDS

AIDS follows the progressive deterioration of the immune system caused by the infection of human immunodeficiency virus, HIV. Early in the disease, cell mediated immunity becomes impaired through a loss of T (thymus derived) cells which express the so-called T4 antigen, a subset of T cells consisting mainly of T helper and inducer cells. T4 cell deficiency may lead to several deficits in cell-mediated immunity, including deficits in NK (natural killer cell), LAK (lymphokine activated killer cell) and cytolytic T cells. There are also B cell (bone marrow derived) defects in AIDS.

AIDS appears to be a progressive disease, although the rate of transition from one phase to another can be variable. These phases have assigned classifications. Asymptomatic individuals infected by HIV exist, sometimes called seropositive, as judged by the presence of circulating antibodies against the virus (M.G. Sarngadharan et al, Science, volume 224, p. 506, 1984). If they have no other markers, they are WR1 stage patients, by the Walter Reed classification (R.R. Redfield et al, New England Journal of Medicine, volume 314, p. 131, 1986). They have been also called pre ARC (ARC = AIDS-related complex), although some of these individuals may never develop ARC symptomatology. Some of these patients may develop lymphadenopathy (WR2) and exhibit the T4 cell deficit (WR3). Next, the ability of lymphocytes to undergo antigen-stimulated proliferation and antigen-stimulated interferon-gamma production decreases and these WR4 patients begin to lose delayed cutaneous hypersensitivity. WR5 patients are usually anergic. They exhibit Herpes Zoster infections, oral candidiasis (thrush), or ARC symptoms which include prolonged fevers, night sweats, fatigue, diarrhea and/or weight loss. This period of ARC is usually also characterized by progressive immune decay. Finally, WR6 individuals experience opportunistic infections constituting "full-blown" or frank AIDS, with 50% of the patients dyeing within 12 months (H.W. Murray et al, New England Journal of Medicine, volume 310, p. 883, 1984).

### BRIEF DESCRIPTION OF THE DRAWINGS

FIGURE 1 is a photograph of a polyacrylamide gel electrophoresis plate showing 7 tracks and bands or zones along each track; and
FIGURE 2 is an illustration of the 2ʹ-5ʹ oligandenylate/RNase L pathway.

### SUMMARY OF THE INVENTION

An inhibitor substance, released or caused to be released by cells infected with human immunodeficiency virus, has been discovered. This inhibitor appears to physically attach to the enzyme RNase L, the terminal product of the 2ʹ-5ʹ oligoadenylate/RNase L pathway, causing the entire immune system to malfunction and allowing the viruses to multiply uncontrollably. Disclosed are techniques of identifying these inhibitors, either directly or indirectly by the absence of RNase L or inactivity of RNase L or the presence of inhibitors of biochemical intermediates in the 2ʹ-5ʹA/RNase L pathway, the inhibitor(s) serving as a marker to detect the presence of HIV or related viruses presenting substantially similar clinical conditions. Prognostic significance of this marker with respect to the development of full-blown AIDS in patients, particularly those having antibodies to HIV in their peripheral blood and in tissues including blood, blood fractions, the cells of transplant organs and cellular extracts are also discussed.

### Derangements in the Cellular "First Line of Defense" (2ʹ-5ʹ oligoadenylate Pathway) in AIDS

Various studies indicate that the 2ʹ-5ʹ oligoadenylate pathway (also referred to in the medical literature as the interferon 2ʹ-5ʹ pathway) is malfunctional in AIDS. This pathway is an integral part of the body's defense system against infections. In AIDS and various antecedent states (e.g., ARC) or LAS (lymphadenopathy syndrome), the proximal enzymes/factors of the pathway are "turned on" or activated but the AIDS virus still multiplies. For example, the interferon level is typically elevated and the enzyme 2-5A synthetase is also elevated. However, the mechanism of these elevations and, in particular the failure of the pathway, at least partially turned on, to inhibit the HIV at least to some degree, was an enigma completely until the studies reported herein.

As the result of my investigations, I have identified an inhibitor, secondary to HIV infection, that exists and that this inhibitor paralyzes the antiviral defense mechanism contributing to full-blown AIDS.

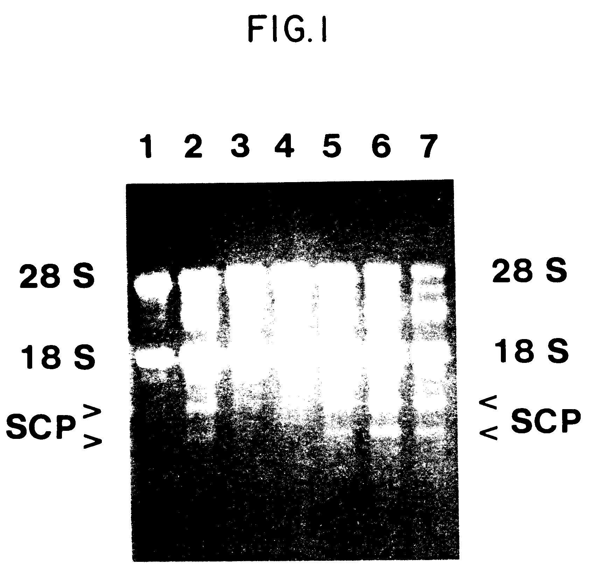

Figure 1 provides a characterization of RNase L in ARC/AIDS patients who rapidly cleared HIV loads in peripheral blood during dsRNA (AMPLIGEN®, a registered trademark of HEM Research, Inc. of Rockville, Maryland, USA) therapy. The presence or absence of activated RNase L in cytoplasmic extracts of patients' peripheral blood mononuclear cells (PBMC) was determined by the ability to generate specific cleavage products (SCP) from 28S and 18S rRNA according to the procedures of K. Kariko and J. Ludwig, Biochem. Biophys. Research Communication, volume 128, p. 695, 1985; D.H. Wreschner et al, Nucleic Acid Research, volume 9, p. 1571, 1981. Ribosomal RNA (containing two molecular species designated 28S and 18S) behaves similarly to viral (e.g., HIV) RNA in being susceptible to cleavage by activated RNase L, but not inactive RNase L. Activated RNase L is characterized by having bound to its molecular structure a preponderance of 2-5A oligomers and a sparse, if any, binding of inhibitor. The results of these studies are shown in Figure 1, a photograph of a polyacrylamide gel electrophoresis plate. Table 1 is a summary of data collected from 10 patients receiving Ampligen therapy.

Extracts of HL929 cells (RNase L minus), used as a source of ribosomal RNA, were incubated for 1 hour in the absence (Lane 1) or presence (Lane 2) of PBMC cytoplasmic extracts from a healthy control subject or from ARC and AIDS patients (all coded) before Ampligen therapy (Lanes 3 and 4 are pretreatment samples from patients 3 and 7 respectively). Lanes 1, 3 and 4 failed to show evidence of any activated RNase L as indicated by bands in the SCP area(s) of the gel.

Before therapy, extracts of all ARC/AIDS patients also showed no activation (no SCP) even when exogenous bioactive oligoadenylates [2'5'-p₃A₃ (10⁻⁸M)] were supplied (gels not shown), which confirmed the non-availability of any functional RNase L molecules in patients' lymphocytes before Ampligen treatment. In contrast, concurrent with Ampligen therapy, progressive in vivo activation of RNase L was observed: in ARC patient 3 (Lane 5 at 8 weeks and Lane 6 at 16 weeks of therapy); in AIDS patient 7 (Lane 7 at 8 weeks) and the various patients of Table 1 (gel not shown). The arrows indicate positions of specific cleavage products (SCP) of RNase L. Only patient number 5 failed to activate RNase L on dsRNA therapy and, as a probable result, he died from a second opportunistic infection.

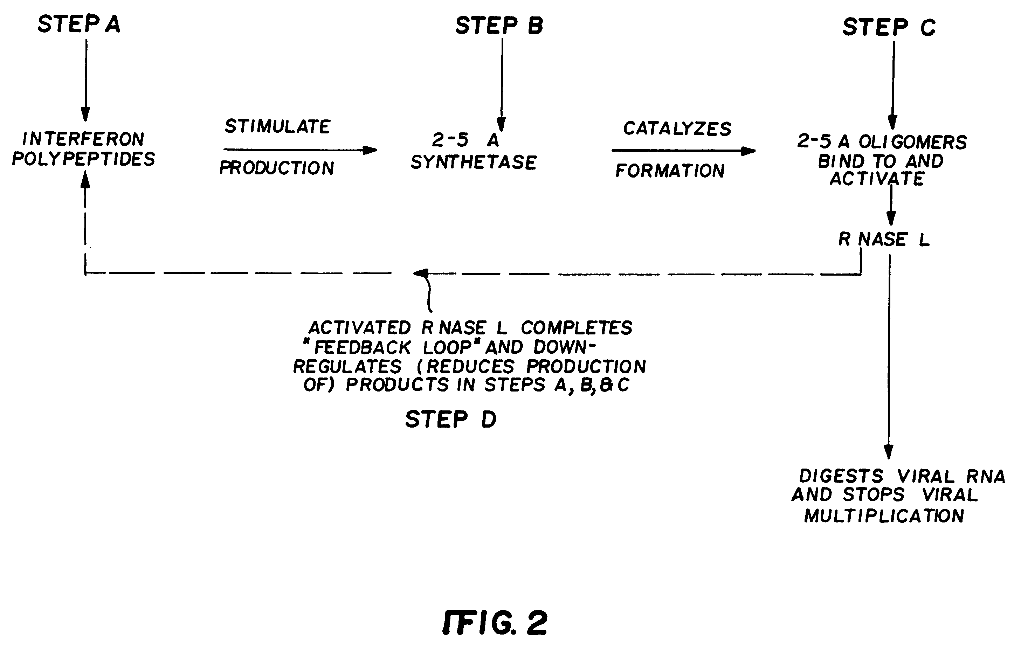

Case 1 - Biological pathway in normal individuals including those who overcame HIV infection is shown in Figure 2.

Case 2 - AIDS and antecedent diseases including ARC, LAS, asymptomatic HIV seropositive individuals who progress to AIDS, etc. (refer to Figure 2) -- Steps [A], [B] and [C] are overfunctioning because RNase L is bound up in inhibitor [step D is non-functional] and cannot complete the pathway; therefore, the pathway cannot properly shut down due to lack of "feedback control" and HIV multiplies even in the face of high concentrations of interferon, 2-5A synthetase and bioactive 2-5 oligomers (particularly active are trimers and tetramers of 2-5A).

Experimental data -- It was first observed that 2-5A oligomers were present in significant concentrations in lymphocytes from untreated individuals throughout the clinical spectrum of AIDS. However, unlike normal individuals (Lane 2 of polyacrylamide gel of Figure 1), RNase L was inactive in untreated ARC and AIDS patients (Lanes 3 and 4). I further observed that, if I obtained inactive RNase L preparations from ARC/AIDS patients and first washed them extensively (before assay), including the use of trichloroacetic acid (TCA), the RNase L preparation would then regain function, i.e., they would catalyze the specific cleavage (designated SCP in gels) or macromolecular RNA and be designated as active. By a series of reconstitution experiments, it was determined that an inhibitor of RNase L had been eluted or broken lose, which, if added back, once again terminated, that is, inhibited, the RNase L reaction.

Evidence available to date indicates that cellular RNase L status (active vs. inactive) constitutes an important prognostic parameter in HIV.

## Claims

1. A method for determining the presence of HIV or another virus causing substantially similar clinical conditions, comprising determining the presence of activated RNase L by measuring the presence of predetermined cleavage products from 28S rRNA and 18S rRNA and bioactive 2' to 5'A oligomers in a patient's cellular extract.

2. The method of claim 1, for determining the presence of occult human immunodeficiency viruses in a human comprising detecting the presence of activated RNase L in human biological fluids or cells.

3. The method of claim 2 in which the biological fluid is blood or a fraction thereof.

4. The method of claim 2, in which the cells are from a skin graft or a transplantable human organ.

5. The method of any one of claims 1 to 4 for determining the projected incidence of HIV in an animal comprising examining an extract of the animal's tissue and determining the presence or absence of activated RNase L molecules, the absence positively indicating the projected presence of HIV.

## Patentansprüche

1. Verfahren zur Bestimmung der Anwesenheit von HIV oder eines anderen Virus, das im wesentlichen ähnliche klinische Zustände hervorruft, **dadurch gekennzeichnet**, daß die Anwesenheit von aktivierter RNase L durch Messen der Anwesenheit von vorherbestimmten Spaltungsprodukten von 28S rRNA und 18S rRNA sowie bioaktiven 2'- bis 5'A-Oligomeren in einem Zellextrakt eines Patienten bestimmt wird.

2. Verfahren nach Anspruch 1 zur Bestimmung der Anwesenheit von okkulten humanen Immundefizienz-Viren in einem Menschen, **dadurch gekennzeichnet**, daß die Anwesenheit von aktivierter RNase L in humanen biologischen Flüssigkeiten oder Zellen festgestellt wird.

3. Verfahren nach Anspruch 2, bei welchem die biologische Flüssigkeit Blut oder eine Fraktion desselben ist.

4. Verfahren nach Anspruch 2, bei welchem die Zellen von einer Hautpfropfung oder einem transplantierbaren menschlichen Organ stammen.

5. Verfahren nach einem der Ansprüche 1 bis 4 zur Bestimmung des geplanten Auftretens von HIV in einem Tier, **dadurch gekennzeichnet**, daß ein Gewebsextrakt des Tieres untersucht und auf Anwesenheit oder Abwesenheit von Molekülen aktivierter RNase L geprüft wird, wobei das Fehlen derselben positiv auf die geplante Anwesenheit von HIV deutet.

## Revendications

1. Procédé pour déterminer la présence de HIV (virus de l'immuno déficience humaine ) ou d'un autre virus provoquant des états cliniques essentiellement similaires , comprenant la détermination de la présence de RNase L (ribonucléase L) activée , par la mesure de la présence de produits prédéterminés de clivage de l'ARNr ( ribosomique ) 28S et la RN'r 18S, et d'oligomères bioactifs 2' vers 5'A dans un extrait cellulaire d'un patient .

2. Procédé selon la revendication 1 , pour déterminer la présence de virus occultes de l'immunodéficience humaine chez un être humain, comprenant la détection de la présence de RNase L (ribonucléase L ) activée dans des fluides biologiques ou des cellules d'origine humaine .

3. Procédé selon la revendication 2 , dans lequel le fluide biologique est du sang ou une fraction de sang .

4. Procédé selon la revendication 2, dans lequel les cellules proviennent d'un greffage de peau ou d'un organe humain transplantable .

5. Procédé selon l'une quelconque des revendications 1 à 4 pour déterminer l'incidence prévue du HIV chez un animal, comprenant l'examen d'un extrait du tissu de l'animal et la détermination de la présence ou de l'absence de molécules activées de ribonucléase L , l'absence indiquant positivement la présence prévue du virus de l'immuno déficience humaine (HIV).
